# EUROPEAN PATENT SPECIFICATION

(11) **EP 2 662 075 B1**
(45) Date of publication and mention of the grant of the patent: **22.02.2023**
(21) Application number: 13002434.2
(22) Date of filing: 07.05.2013
(51) Int. Cl.: A61K 9/16, A61K 31/365, A61K 31/4184, A61K 31/4985, A61K 31/506, A61K 31/7042, A61K 47/44

(54) **Taste masking compositions of praziquantel**
Geschmacksmaskierungszusammensetzungen von Praziquantel
Compositions de masquage de goût de praziquantel

(30) Priority: 08.05.2012 HU 1200265
(43) Date of publication of application: 13.11.2013
(73) Proprietor: Lavet Gyogyszergyarto es Szolgaltato Kft., 1161 Budapest (HU)
(72) Inventor: LACZAY, Péter, H-1161 Budapest (HU)
(74) Representative: Szentpéteri, Zsolt

(56) References cited:
- EP-A1- 1 362 583
- EP-A1- 1 566 173
- WO-A1-2010/063387
- WO-A2-2007/073911
- GB-A- 2 403 407
- US-A1- 2006 228 399

## Description

The present invention relates to improvements in the oral formulations of the anthelmintic praziquantel used for control and treatment of endoparasitic infestations in warm-blooded animals in particular in companion animals such as cats, dogs and horses and methods of producing the formulations.

The primary attributes of a successful pharmaceutical product for companion animals remain efficacy and safety of the active ingredient. However, the importance of palatability has significantly increased during the past several years because it markedly affects convenience and compliance.

It can be a rather challenging task for a pet owner to administer an oral product to cats or dogs as the animals mostly do not take their medicine willingly, especially if it is badly tasting or smelling. Their acute sense of smell will often warn them that their owner is approaching with an unpleasant drug product even when it is hidden in a treat or the food. Administering, bad-tasting medicines to a horse also offers several challenges. Horses are intelligent animals, and will learn after the first effort that the tube inserted in their mouth has an unpleasant flavour. Therefore, palatability is one of the main factors to be considered in development of oral formulations for companion animals.

Examples of such unpleasant medicines are anthelmintic veterinary formulations containing the active ingredient praziquantel (2-(cyclohexylcarbonyl)-1,2,3,6,7-11b-hexahydro-4H-pyrazino[2,1-a]isoquinoline-4-one). Praziquantel is a well known anthelmintic agent, widely used in both human and animal medicines. In animal health it is used against cestodes and trematodes, whereas in human pharmaceuticals praziquantel is the drug of choice for treatment of all forms of schistosomiasis.

Praziquantel has a very bitter taste and a bad smell, a consequence of which it is refused virtually by all cats and dogs offered the drug without some type of taste masking. The simplest way to try to overcome the bitter taste of praziquantel is to add a well-accepted flavour to the composition. However, the simple addition of a flavour may not be sufficient because this does not mask the taste of very bitter drugs and also does not cover odours. Cats are especially sensitive to bad taste and odour and are not tricked as easy as dogs by simply adding a flavour. Other methods to achieve taste masking may include coating, complexation or embedding in taste masking agents. The most often used and easiest technique to mask the bitter taste of human medicines is coating because it encloses the drug and therefore also masks the odour. However, the animals may crush the coating during chewing and as a result, perceive the bitter taste and refuse to swallow the drug product. A better result is achieved if microparticles of granules of the bitter drug are coated with different polymers or by complex formation with suitable carriers, such as cyclodextrins by co-precipitation or by kneading.

Nearly all of these processes require solvents, i.e. water, organic solvents or mixtures. The use of organic solvents may lead to environmental problems, solvent residues and excessive cost of recovery. Furthermore, instability of the active ingredient in these solvents may occur, and aqueous solvents generally prolong the duration of processes.

Several thermal techniques such as melt granulation, hot-melt extrusion, hot-melt coating and spray congealing may also show promising results for taste masking of bitter drugs. These methods are based on the use of suitable hydrophobic carriers, mostly lypophilic waxes with a rather low melting temperature. Because of the hydrophobic nature of the lipid carriers, the release of drug substances is mostly delayed. This may affect the bioavailability of the drugs especially in case of those substances for which the dissolution is the absorption rate limiting factor. As regards the biopharmaceutics properties, praziquantel belongs into this class of drugs (Class II in the Biopharmaceutics Classification System).

Various methods of taste masking praziquantel have been described. US Patent No. 7,348,027 provides a method of possible taste masking by mixing praziquantel, pyrantel embonate and febantel with artificial beef and yeast components and subjected to a first compression. The resulting rough tablet is then ground to increase the density of the material which is then subjected to a second compression to form a final, self-take tablet. This method of taste masking is based on adding a well-accepted flavour to the composition without applying an integral coating to the particles.

WO 2005/081612 describes a solid pharmaceutical composition for veterinary use, where such composition includes a compressed nucleus which contains the active ingredients of unpleasant taste, such as praziquantel and pharmaceutically accepted excipients, as well as a coat which includes flavourings and compression co-adjuvants.

According to this invention the coat is compressed onto a nucleus that is produced by moist granulation without applying an integral coating to the particles.

EP Patent No. 1,566,173 provides a method of taste masking praziquantel in which the anthelmintic agent is adsorbed to the inner surface of an excipient having a porous structure with particular surface area providing an efficient taste- and odour-masking effect as well as a sustained release profil. Using charcoal as excipient, a good taste masking and sustained release of praziquantel was achieved. This method of taste masking is based on adsorption the bitter compound to a porous excipient.

GB Patent No. 2,403,407 discloses a method of taste masking where praziquantel and fenbendazole are wet granulated with a taste-masking polymer, sweetener and sweet excipients. The taste-masking polymer, sweetener and sweet excipients fill up the intergranular mass of the composition, thereby providing a sweeter taste to it. This method of taste masking is based on wet granulation the bitter compound with a taste-masking polymer.

EP Patent No. 1,362,583 relates to masking the taste of praziquantel and epsiprantel by microencopsulation comprising a dispersion of the active ingredients surrounded by an impermeable capsule wall. This method of taste masking is based on microencapsulation of the bitter compound using a suitable capsule wall-forming material.

WO 2007/073911 provides a method of taste masking powders that are to be inhaled or administered orally by coating the microparticles of the active substance, such as praziquantel with a wax dissolved in an organic solvent and then spray dried to obtain particles between 1 and 40 µm. Suitable solvents according to the invention are aromatic or aliphatic hydrocarbons which are liquid at room temperature, short-chain alcohols, short-chain ketones, carboxylic acids, ethers, esters, heterocyclic amines. Particularly preferred solvents are n-heptane and methylcyclohexane. Accordingly, this method of taste masking is based on wax coating by spray drying using organic solvents.

EP 2008/065335 is concerned with an anthelmintic paste, comprising praziquantel and a macrocyclic lactone as active ingredients. According to the invention taste masking of praziquantel is achieved by inclusion complexation with cyclodextrins.

WO 2008/148484 describes a method of taste masking praziquantel and some other pharmacologically active substances by melt extrusion with thermoformable materials including polymers, lipids, surfactants, macrogols, sugars, sugar alcohols and combination thereof. The extrudates of the invention have a strand diameter of 0.5 mm or less which is said to be suitable for concealing the taste of ingredients with an unpleasant taste. Accordingly, this method of taste masking is based on hot-melt extrusion.

WO 2010/063387 also relates to extrudates containing at least one pharmaceutically active substance in the form of needles, wherein the ratio of the particle size of the needle-shaped pharmaceutically active substance to the strand diameter is 1:15. According to this invention extrudates characterised by their strand diameter are produced that can be subsequently cut into pieces by appropriate chopper.

EP Patent No. 1,675,474 relates to chewable veterinary compositions of parasiticidal agents including praziquantel by extrusion of the active ingredients with partially gelatinized starch and meat flavouring comprising a high fat content at temperatures not exceeding 40°C.

As can be seen from data above, the methods applied for taste masking of praziquantel until now include dry and wet granulation, adsorption to porous substances, polymer and wax coating using organic solvents, microencapsulation and extrusion.

We have now found that the extremely bitter taste of praziquantel may be masked by embedding the drug substance into a suitable lipid. The praziquantel particles of integral lipid coatings are substantially insoluble in water as a consequence of which do not release the bitter praziquantel in the wet environment of the mouth. Because of the hydrophobic nature of the lipids it was expected that the lipid coating of the particulate praziquantel will also delay the release of the active in the gastrointestinal tract as described e.g. by Jannin et al. (Jannin, V. et al: Influence of poloxamers on the dissolution performance and stability of controlled-release formulations containing Precirol ATO 5. International Journal of Pharmaceutics, 309, 6-15.). For veterinary products, mainly for cats and dogs, this is not reasonable as the gastrointestinal transit time is less in companion animals than in humans.

Therefore, it is suprising and was absolutely not predictable that the formulations according to the present invention containing the lipid-coated praziquantel could lead to the same level of bioavailability in terms of bioequivalence as the administration of similar formulations containing non-coated praziquantel. The formulated coated particles, while not releasing the bitter praziquantel in the wet environment of the mouth, break down upon contact with the gastrointestinal fluid, thus allowing rapid dispersion and dissolution in the gastrointestinal tract. Thus, the present invention provides highly palatable, easy to administer oral anthelmintic compositions that are at least as efficacious and safe as conventional oral praziquantel formulations.

Thus according to one aspect the present invention provides a composition comprising particles of praziquantel with an integral coating of a lipid or a mixture of lipids which are insoluble in water and which serve to mask to extremely bitter taste of praziquantel upon oral administration but which disperse or dissolve on contact with gastrointestinal tract and thus do not influence the bioavailability of the drug substance.

According to a further aspect, the present invention provides compositions for oral administration of praziquantel in cats and dogs which are substantially free of the bitter taste associated with praziquantel and comprise a dispersion of the lipid-coated particles of praziquantel in suitable vehicle. According to a yet further aspect, the invention provides a method of masking the bitter taste of praziquantel which comprises coating praziquantel with a suitable lipid or a mixture of lipids and incorporating the lipid-coated particles into oral formulations, for example into tablets, oral pastes or gels.

In order to effectively mask the bitter taste of praziquantel the mean diameter of the particles should be less than 100 µm.

The melting point of the lipid used should be sufficiently high to prevent melting of the coated particles in the mouth, thereby leading to release of the bitter tasting active substance, but not so high that praziquantel itself melts and/or becomes chemically degraded during the coating process. Thus the lipid or mixture of lipids for use in the present invention will have a melting point from 40°C to 80°C and preferable from 50°C to 70°C.

Suitable lipids for the coating of praziquantel particles include fatty acids or monohydric alcohols thereof, fixed oils, fats, waxes, sterols, phospholipids either singly or in mixture. The lipid may, for example, be a high molecular weight (C₁₀-C₃₀) straight chain saturated or unsaturated aliphatic acid, such as palmitic or stearic acid, glycerol mono-, di- or triesters of C₁₀-C₃₀ fatty acids, such as glycerol dibehenate, glycerol distearate, glycerol trilaurate, glycerol myristate, glycerol tripalmitate or glycerol tristearate and mixtures of glycerol mono-, di- or triesters such as, for example, glycerol palmitostearate. Mention may also made of waxes, especially those having 30 to 60 carbon atoms, such as cetyl palmitate. Mention may furthermore be made of partially hydrogenated vegetable or animal oils such as hardened palm oil or hardened edible tallow, and high molecular weight (C₁₀-C₃₀) straight chain aliphatic alcohols such as stearyl alcohol or cetyl alcohol. Particularly preferred examples are glycerol distearate and cetyl palmitate.

The lipid-coated particles according to the invention will contain 5 to 80%, preferably 10 to 50%, more preferably 20 to 40% praziquantel on a weight-to-weight (w/w) basis.

The lipid-coated particles of the invention will generally have a diameter of less than 500 µm, and preferably less than 300 µm. Coated particles with a diameter in the range of 1-300 µm, e.g. 40-200 µm are preferred. Control of the particle size is necessary to ensure that the subsequently formulated product does not induce a gritty feel in the mouth. Particles having a diameter of less than about 200 µm are preferred in this respect.

The lipid-coated particles of the invention may be prepared by atomising a dispersion of praziquantel in a molten lipid and cooling the particles hereby obtained and such a process constitutes a further feature of the invention. The dispersion may be prepared by adding particulate praziquantel to the molten lipid or mixture of lipids or alternatively mixing the ingredients of the dispersion together in the solid state and melting the lipid or mixture of lipids. The particulate praziquantel can be dispersed in the molten lipid using conventional techniques, for example, using a high shear mixer. Generally, the temperature of the molten lipid should be 20-30°C above its melting point. In general, the lipid or lipid mixture used for coating of praziquantel particles should have a melting point within the range of 40 to 100°C, preferably 50 to 70°C, and the temperature of the molten lipid will be 20 to 40°C above its melting point. Atomising techniques which may be applied include the use of conventional atomisers such as rotary atomisers, pressure nozzles and sonic nuzzles. The use of a two-fluid pneumatic nozzle atomiser fitted in a standard spray congealing/chilling apparatus is particularly convenient.

In the atomisation process, the molten lipid dispersion will generally be supplied to the atomiser head at a temperature in the range of 70°C to 110°C, preferable 80°C to 100°C, the precise temperature depending on the particular lipid used. The atomising gas supplied to the nozzle may be air or an inert gas such as nitrogen. The atomising pressure is desirably controlled in order to produce particles of the preferred size.

The coated particles may be solidified and collected by conventional techniques. The coated particles may conveniently be solidified by applying a stream of cool air or dry nitrogen to the spray chamber at a temperature between 0°C to 30°C, preferably 5°C to 15°C. The lipid-coated particles are collected as a free flowing powder using either a cyclone separator or a bag filter. The particles are spherical in shape and have a particle size preferably less than 300 µm and more preferably in the range of 40 to 200 µm. The lipid-coated particles of the invention may be incorporated into a pharmaceutical composition for oral administration, using further pharmacologically active ingredients and physiologically acceptable carriers or excipients.

The compositions according to the invention may for example include tablets, granules, powders, suspensions, oral pastes and gels. Tablets and oral pastes or gels represent particularly preferred dosage forms.

Accordingly, the present invention relates in one embodiment to an antiparasitic oral formulation, preferably a tablet or oral paste or gel, comprising
- a veterinarily effective amount of praziquantel as anticestodal agent in the lipid-coated form,
- veterinarily effective amounts of one or more antinematodal and/or endectoidal agent selected from benzimidazoles, tetrahydropyrimidins, avermectins, milbemycins and derivatives thereof, which are active against roundworms and/or ectoparasites of cats and dogs,
- physiologically acceptable excipients.

The amount of praziquantel in the oral formulations is preferably in the range of 0.1% to 50% by weight, more preferably 0.5% to 25% by weight, based on the entire formulation.

The oral antiparasitic formulations of the present invention comprise veterinarily effective amounts of further active ingredients which may be selected from benzimidazoles tetrahydropyrimidines, avermectins, milbemycins and derivatives thereof. Preferred, non-limiting examples of actives used in combination with the lipid-coated praziquantel according with the invention are: Febantel (CAS No: 58306-30-2); Pyrantel (CAS No: 22204-24-6); Ivermectin (CAS No. 70288-86-7); Doramectin (CAS No. 117704-25-3); Moxidectin (CAS No. 113507-06-5); Selamectin (CAS No. 220119-17-5); Emamectin (CAS No. 119791-41-2); Eprinomectin (CAS No. 123997-26-2); Milbemycin D (CAS No. 77855-81-3); Abamectin (CAS No. 71751-41-2); Milbemycin oxime (CAS No. 129496-10-2); Nemadectin (CAS No. 102130-84-7) and derivatives thereof, in free form or in the form of a physiologically acceptable salt.

The antinematodal compounds are present preferably in the oral antiparasitic formulations of the invention in the range of 0.01 to 50% by weight, more preferably from 0.05 to 30% by weight, based on the entire weight.

The oral antiparasitic compositions may be formulated using conventional pharmaceutically acceptable excipients. Thus for example tablets may be prepared by using binding agents (e.g. pregelatinised starch, polyvinyl pyrolidine or hydroxypropyl methyl cellulose), fillers (e.g. lactose, microcrystalline cellulose or calcium hydrogen phosphate), lubricants (e.g. magnesium stearate, talc or colloidal silica), disintegrants (e.g. starch or sodium starch glycolate) or wetting agents (e.g. sodium laurel sulphate). The tablet formulations of the present invention in addition, contain one or more attractive, physiologically acceptable natural or synthetic flavourings. Preferred flavouring agents are artificial beef flavour, liver powder and brewer's yeasts. The most preferred artificial beef flavour is supplied by Pharma Chemie Inc, 1877 Midland Street, Syracuse, Nebr. 4866. The preferred flavour is identified as PC-0125. The flavour is a hydrolyzed vegetable protein and so is free of Bovine Spongiform Encephalopathy (BSE) and Hoof and Mouth Disease (HMD) contamination. The flavouring agents are present preferably in the tablet formulations of the invention in the range of 1.0 to 60% by weight, more preferably from 5.0 to 30% by weight, based on the entire weight.

The oral paste or gel formulations of the present invention are water-based suspensions which are devoid of any organic solvent dissolving the lipid-coated praziquantel and/or any further antiparasitic agent used in the formulation. The water content of the formulations is, for example ≥30% by weight, preferably ≥50% by weight, and in particular ≥60% by weight, based on the weight of the entire formulation. The aqueous oral paste or gel formulations of the present invention may advantageously contain an orally acceptable solute at relatively high concentration since this assists in maintaining the taste-masking properties of the lipid coating. Thus, for example the aqueous medium contain a sugar, e.g. sucrose preferably in the concentration range of 40 to 85% by weight.

The oral paste or gel formulation of the present invention contains one or more pharmacologically acceptable thickeners. Suitable thickeners useful in the oral paste or gel formulations of the present invention include in principle all thickeners being acceptable for oral application in animals. Examples of suitable thickeners are xanthan gum, polyvinyl pyrrolidones, polyacrilates such as carbopols, cornstarch, microcrystalline cellulose, hydroxyethyl cellulose, silicon dioxide or combinations thereof. Preferred thickeners are xanthan gum, carbopols, microcrystalline cellulose, polyvinyl pyrollidone or a mixture of two or three thereof. The thickener is present preferably in the oral paste or gel formulations of the present invention in the range of 0.1 % to 10% by weight, more preferably from 1% to 5% by weight, based on the entire formulation. The antiparasitic oral paste or gel formulation of the present invention, in addition, may contain further pharmacologically acceptable excipients, for example humectants (e.g. glycerol) preservatives (e.g. benzyl alcohol, benzoic acid, benzoates or p-hydroxibenzoates, pH-adjusting agents (bases or acids), binders, fillers, surface active or dispering agents. Said pharmacologically acceptable ingredients are known to those skilled in the art of veterinary formulation technology. The oral paste and gel formulations of the present invention also contain veterinarily acceptable attractive flavourings. Suitable flavouring agents within the compositions of the invention are, for example, artificial beef flavour, food extracts such as desiccated liver or malt extract and honey flavours.

The pharmaceutical compositions of the invention may be prepared according to conventional techniques well known in the pharmaceutical industry.

Thus for example tablets may be prepared by direct compression of a dry admixture of the lipid-coated praziquantel, with further active substances and excipients or by wet granulation. Oral paste or gel formulations may be obtained by dispering the lipid-coated particles of praziquantel and the further active ingredients in suitable aqueous vehicles.

The compositions for use according to the present invention may, if desired, be presented in a pack or dispenser device which may contain one or more unit doses. The pack may for example comprise metal or plastic foil, such as blister pack.

The tablet and oral paste or gel formulations of the present invention are suitable for controlling pathogenic endoparasites in warm-blooded animals, such as, for example, companion animals, in particular dogs and cats. They are effective against all or individual development stages of the parasites including cestodes, nematodes, trematodes and acanthocephala.

The basis of efficacy is an adequate bioavailability of the active ingredients in the target animals. The plasma bioavailability of a tablet formulation containing the lipid-coated praziquantel according to Example 3 of the present invention was studied in Beagle dogs in comparison to an authorized reference product. The plasma concentrations of praziquantel were determined by a validated LC/MS method. The bioequivalence of the test and reference formulations was assessed using a SAS 9.2 software package.

Moreover, the oral formulations according to the present invention have an improved palatability meaning the voluntary acceptance or ingestion of the formulations by warm-blooded animals in particular by dogs and cats. The acceptance of the tablet and oral paste/gel formulations containing lipid-coated praziquantel and suitable further ingredients according to the present invention was tested on dogs and cats, respectively.

### Examples

The following examples illustrate the present invention.

### Example 1: Lipid coating of praziquantel

Glycerol distearate (8.0 kg) was melted in a stainless steel vessel and raised to a temperature of 85°C. Praziquantel (2.0 kg) with a mean particle size <100 µm was added to the molten lipid. The molten dispersion was mixed with a high shear mixer to give a homogeneous suspension and pumped to a spray congealing apparatus. The mixture was atomised using a pressure nozzle atomisation system using atomising gas rates of 5-7 kg/h. The atomised droplets were chilled using air fed into the spray chamber at a temperature of 5 to 10°C and the solid particles were collected in a cyclone separator. The lipid-coated praziquantel comprised spherical particles with a mean particle size of 90 µm.

### Example 2: Lipid coating of praziquantel

Cetyl palmitate (7.25 kg) was melted in a stainless steel vessel while raising the temperature to 90°C. To this was added praziquantel (2.75 kg) with a particle size of <100 µm. The molten dispersion was subjected to high shear mixing to obtain a homogenous suspension and this mixture was spray chilled as described in Example 1, to give a lipid-coated product having a similar particle size and shape.

### Example 3: Tablet formulation

Cetyl palmitate coated praziquantel was blended with pyrantel embonate, febantel, artificial beef flavour and other ingredients in a rotary blender. The resultant mix was compressed into tablets using a suitable tablet press and suitable punches.

| *Direct Compression Tablets* | *Amount (% w*/*w)* |
|---|---|
| Lipid coated Praziquantel* | 21.4 |
| Febantel | 17.6 |
| Pyrantel embonate | 16.9 |
| Artificial beef flavour | 21.5 |
| Pregelatinised starch | 7.6 |
| Sodium starch glycolate | 7.0 |
| Lactose | 7.0 |
| Colloidal silica | 1.0 |
| * According to Example 2 | |

### Example 4: Paste formulation

Glycerol distearate coated praziquantel, moxidectin and the excipients were dispersed consecutively in an aequeous xanthan gel and the dispersion was stirred until a homogeneous paste was formed.

| *Oral Paste* | *Amount (% w*/*w)* |
|---|---|
| Lipid coated Praziquantel* | 4.0 |
| Moxidectin | 0.05 |
| Xanthan qum | 0.5 |
| Lactose | 26.7 |
| Saccharose | 33.4 |
| Wheat starch | 2.0 |
| Artificial honey | 2.0 |
| Methyl parahydroxybenzoate | 0.05 |
| Purified water | ad 100.0% |

| | |
|---|---|
| * According to Example 1 | |

### Example 5: In vivo bioavailability

The *in vivo* bioavailability of praziquantel used in the lipid-coated form according to Example 2 of the invention and administered as a tablet formulation according to Example 3 of the present invention was determined in comparison to a reference formulation containing the same amount of praziquantel (50 mg/tablet) but in non-coated form. Both formulations were administered at a dose of 5 mg praziquantel per kg of body weight in a cross-over design. Geometric means of the measured/calculated kinetic parameters were found as follows:

| | ***Measured*/*calculated kinetic parameters of praziquantel*** | |
|---|---|---|
| | ***Test formulation of the invention*** | ***Reference formulation*** |
| Cₘₐₓ (ng/ml) | 308.3 | 325.6 |
| Tₘₐₓ (h) | 1.9 | 1.8 |
| AUC (ng·h/ml) | 921.1 | 984.0 |

| | | |
|---|---|---|
| Cₘₐₓ = maximum plasma concentration; Tₘₐₓ = time to reach the maximum concentration; AUC = area under the curve | | |

Based on the statistical evaluation of the relevant pharmacokinetic parameters the test formulation containing the lipid coated praziquantel was found to reliably fulfil the bioequivalence requirements.

### Example 6: Palatability (acceptance) test

129 male and female dogs of different breeds and age were tested. The testing person offered to each dog a single dose of the test formulation which was adapted to the body weight of the animal. In the case of tablet formulations of the present invention, in a first instance the tablet was offered by hand for 1 minute. If the dog does not take the formulation it was offered the dog in his empty bowl. The dog had again 1 minute to take the formulation. If not, it was placed into his/her mouth. If one of the three abovementioned offers leads to the dog willingly eaten the tablet, this is evaluated as acceptance of the tablet. If the dog spits it out it is reported as not accepted.

A similar test was carried out with the oral paste/gel formulations of the present invention on 34 cats. The tested formulations were filled in graduated oral syringes. Throughout the study conduct, 1 ml of all tested oral paste/gel formulations were offered to each cat. First, a small amount of paste was applied on the lips/nose of the animal and if the cat spontaneously licked and swallowed the paste the complete dose was administered by this way. When the paste was not spontaneously licked, the total dose was administered by interesting the syringe in the cat's mouth. The acceptability was assessed based on the cat's behaviour and scored as follows:
score 2 = swallowed all;
score 1 = swallowed part;
score 0 = entirely rejected/spat it all out.

| ***Tablet formulation*** | ***Flavour*** | ***Total acceptance by dogs (%)*** |
|---|---|---|
| Tablet containing lipid-coated Praziquantel, Febantel and Pyrantel embonate | Pig, liver powder, brewer's yeast | 91.6 |
| Tablet containing lipid-coated Praziquantel, Febantel and Pyrantel embonate | Artificial beef | 95.4 |
| Tablet containing lipid-coated Praziquantel, Febantel and Pyrantel embonate | Artificial liver flavour | 88.7 |
| Tablet containing lipid-coated Praziquantel, Febantel and Pyrantel embonate | Pig liver powder, brewer's yeast | 90.9 |

| ***Paste formulation*** | ***Flavour*** | ***Total acceptance by cats (%)*** | | |
|---|---|---|---|---|
| | | *Score* 2 | *Score* 1 | *Score* 0 |
| Paste containing lipid-coated Praziquantel and Moxidectin | Artificial beef | 61.8 | 29.4 | 8.8 |
| Paste containing lipid-coated Praziquantel and Moxidectin | Artificial honey | 58.8 | 32.4 | 8.8 |
| Paste containing lipid-coated Praziquantel and Pyrantel embonate | Artificial beef | 67.6 | 26.5 | 5.9 |
| Paste containing lipid-coated Praziquantel and Pyrantel embonate | Artificial honey | 64.7 | 26.5 | 8.8 |

## Claims

1. A composition which is substantially free of the bitter taste associated with praziquantel and comprises praziquantel in particulate form coated with a lipid or mixture of lipids which serve to mask the extremely bitter taste of praziquantel upon oral administration wherein
a) the lipid or lipid mixture comprises one or more straight chain aliphatic carboxylic acids having from 10 to 30 carbon atoms
b) the concentration of the lipid or lipids used for lipid coating ranges from 20 to 95% w/w
c) the melting point of the lipid or lipids ranges from 40 to 80 °C
d) the particles have diameters in the range of 1 to 300 µm
e) organic solvents are excluded from the formulation.

2. A composition according to Claim 1 wherein the lipid or lipid mixture is the cetyl or glycerol ester of stearic acid or palmitic acid.

3. A composition according to Claim 1 wherein the melting point of the lipid or lipid mixture ranges from 50 to 70 °C.

4. A composition according to Claim 1 wherein the praziquantel content of the coated particles ranges from 5 to 80% w/w.

5. A composition as claimed in Claim 4 wherein the praziquantel content of the coated particles is in the range from 20 to 40% w/w.

6. A process for the preparation of a composition according to Claim 1 which comprises dispersing particulate praziquantel in a molten lipid, atomising the dispersion and then cooling and collecting the coated particles, thereby obtained.

7. A pharmaceutical composition for oral administration to warm-blooded animals in particular to cats, dogs and horses, comprising
a) a veterinary effective amount of composition as claimed in Claim 1,
b) veterinary effective amounts of one or more antinematodal agents selected from benzimidazoles, tetrahydropyrimidines or macrocyclic lactones such as avermectins, milbemycins and derivatives thereof,
c) one or more physiologically acceptable excipients.

8. A pharmaceutical composition as claimed in Claim 7 in the form of tablets and oral pastes or gels.

9. A pharmaceutical composition according to Claim 7 containing praziquantel in the range of 0.5 to 25% w/w, based on the entire formulation.

10. A pharmaceutical composition according to Claim 7, wherein the benzimidazole agent is febantel or fenbendazole and the tetrahydropyrimidine compound is pyrantel embonate.

11. A pharmaceutical composition according to Claim 7, wherein the macrocyclic lactone is of formula: 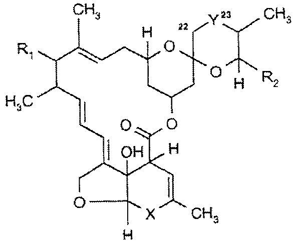 wherein X is -C(H)(OH)-; -C(O)-; or -C(=N-OH)-; Y is -C(H₂)-; =C(H)-; -C(H)(OH)-; or - C(=N-OCH₃)-; R-i is hydrogen or one of radicals 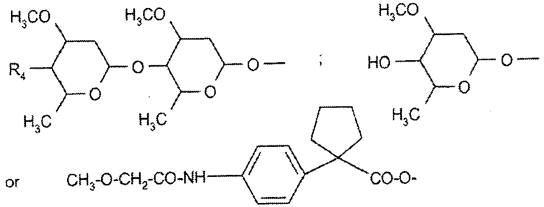 R₄ is hydroxyl, -NH-CH₃ or-NH-OCH₃; and R₂ is hydrogen, -CH₃, -C₂H₅, -CH(CH₃)₂, - CH(CH₃)-C₂H₅, -C(CH₃)=CH-CH(CH₃)₂ or cyclohexyl; and if the bond between atoms 22 and 23 represents a double bond the carbon atom in 23-position is unsubstituted so that Y is =C(H)-, or if is the bond between atoms 22 and 23 is a single bond the carbon atom in 23-position is unsubstituted or substituted by hydroxy or by the group =N-0-CH₃ so that Y is -C(H₂)-; -C(H)(OH)-; or -C(=N-OCH₃)-; in free form or in the form of a physiologically acceptable salt.

12. A pharmaceutical composition according to Claim 7 containing one or more antinematodal agents in the range of 0.01 to 50% w/w, based on the entire formulation.

## Patentansprüche

1. Zusammensetzung, die weitgehend frei von dem mit Praziquantel assoziierten bitteren Geschmack ist und Praziquantel in teilchenförmiger Form, die mit einem Lipid oder einer Mischung von Lipiden, das bzw. die dazu dient, den extrem bitteren Geschmack von Praziquantel bei oraler Verabreichung zu maskieren, überzogen ist, umfasst, wobei
a) das Lipid bzw. die Lipidmischung eine oder mehrere geradkettige aliphatische Carbonsäuren mit 10 bis 30 Kohlenstoffatomen umfasst,
b) die Konzentration des Lipids bzw. der Lipide, das bzw. die für den Lipidüberzug verwendet wird bzw. werden, im Bereich von 20 bis 95 % w/w liegt,
c) der Schmelzpunkt des Lipids bzw. der Lipide im Bereich von 40 bis 80 °C liegt,
d) die Teilchen Durchmesser im Bereich von 1 bis 300 pm aufweisen,
e) organische Lösungsmittel aus der Formulierung ausgeschlossen sind.

2. Zusammensetzung nach Anspruch 1, wobei es sich bei dem Lipid bzw. der Lipidmischung um den Cetyl- oder Glycerinester von Stearinsäure oder Palmitinsäure handelt.

3. Zusammensetzung nach Anspruch 1, wobei der Schmelzpunkt des Lipids bzw. der Lipidmischung im Bereich von 50 bis 70 °C liegt.

4. Zusammensetzung nach Anspruch 1, wobei der Praziquantelgehalt der überzogenen Teilchen im Bereich von 5 bis 80 % w/w liegt.

5. Zusammensetzung nach Anspruch 4, wobei der Praziquantelgehalt der überzogenen Teilchen im Bereich von 20 bis 40 % w/w liegt.

6. Verfahren zur Herstellung einer Zusammensetzung nach Anspruch 1, bei dem man teilchenförmiges Praziquantel in einem geschmolzenen Lipid dispergiert, die Dispersion zerstäubt und dann die dadurch erhaltenen überzogenen Teilchen abkühlt und sammelt.

7. Pharmazeutische Zusammensetzung zur oralen Verabreichung an Warmblüter, insbesondere an Katzen, Hunde und Pferde, umfassend
f) eine veterinärmedizinisch wirksame Menge einer Zusammensetzung nach Anspruch 1,
g) veterinärmedizinisch wirksame Mengen eines oder mehrerer antinematodaler Mittel, die aus Benzimidazolen, Tetrahydropyrimidinen oder makrocyclischen Lactonen wie Avermectinen, Milbemycinen und Derivaten davon ausgewählt sind,
h) einen oder mehrere physiologisch unbedenkliche Hilfsstoffe.

8. Pharmazeutische Zusammensetzung nach Anspruch 7 in Form von Tabletten und oralen Pasten oder Gelen.

9. Pharmazeutische Zusammensetzung nach Anspruch 7, enthaltend Praziquantel im Bereich von 0,5 bis 25 % w/w, bezogen auf die gesamte Formulierung.

10. Pharmazeutische Zusammensetzung nach Anspruch 7, wobei es sich bei dem Benzimidazol-Mittel um Febantel oder Fenbendazol handelt und es sich bei der Tetrahydropyrimidinverbindung um Pyrantelembonat handelt.

11. Pharmazeutische Zusammensetzung nach Anspruch 7, wobei das makrocyclische Lacton die Formel: 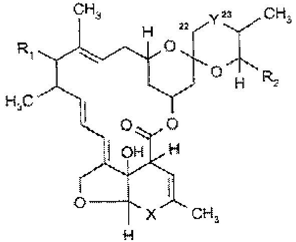 aufweist, wobei X für -C(H)(OH)-; -C(O)- oder -C(=N-OH)- steht; Y für -C(H2)-; =C(H)-; -C(H)(OH)- oder -C(=NOCH3)- steht; R-i für Wasserstoff oder einen der Reste 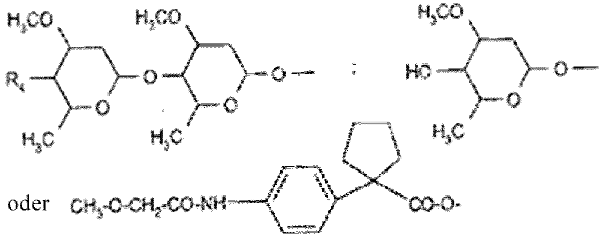 steht; R4 für Hydroxyl, -NH-CH3 oder -NH-OCH3 steht und R2 für Wasserstoff, -CH3, -C2H5, -CH(CH3)2, -CH(CH3)-C2H5, -C(CH3)=CH-CH(CH3)2 oder Cyclohexyl steht und dann, wenn die Bindung zwischen den Atomen 22 und 23 eine Doppelbindung repräsentiert, das Kohlenstoffatom in der 23-Position unsubstituiert ist, so dass Y für =C(H)-steht, oder dann, wenn die Bindung zwischen den Atomen 22 und 23 eine Einfachbindung ist, das Kohlenstoffatom in der 23-Position unsubstituiert oder durch Hydroxy oder durch die Gruppe =N-O-CH3 substituiert ist, so dass Y für -C(H2)-; -C(H) (OH)- oder -C(=N-OCH3)- steht; in freier Form oder in Form eines physiologisch unbedenklichen Salzes.

12. Pharmazeutische Zusammensetzung nach Anspruch 7, enthaltend ein oder mehrere antinematodale Mittel im Bereich von 0,01 bis 50 % w/w, bezogen auf die gesamte Formulierung.

## Revendications

1. Composition qui est sensiblement exempte du goût amer associé au praziquantel et comprend du praziquantel sous forme particulaire revêtue par un lipide ou un mélange de lipides qui servent à masquer le goût extrêmement amer du praziquantel lors d'une administration orale,
a) le lipide ou mélange de lipides comprenant un ou plusieurs acides carboxyliques aliphatiques à chaîne linéaire possédant de 10 à 30 atomes de carbone
b) la concentration du lipide ou des lipides utilisés pour un revêtement de lipide se situant dans la plage de 20 à 95 % p/p
c) le point de fusion du lipide ou des lipides se situant dans la plage de 40 à 80 °C
d) les particules possédant des diamètres dans la plage de 1 à 300 pm
e) des solvants organiques étant exclus de la formulation.

2. Composition selon la revendication 1, le lipide ou le mélange de lipides étant l'ester de cétyle ou de glycérol d'acide stéarique ou d'acide palmitique.

3. Composition selon la revendication 1, le point de fusion du lipide ou du mélange de lipides se situant dans la plage de 50 à 70 °C.

4. Composition selon la revendication 1, la teneur en praziquantel des particules revêtues se situant dans la plage de 5 à 80 % p/p.

5. Composition selon la revendication 4, la teneur en praziquantel des particules revêtues se situant dans la plage de 20 à 40 % p/p.

6. Procédé pour la préparation d'une composition selon la revendication 1 qui comprend la dispersion de praziquantel particulaire dans un lipide fondu, l'atomisation de la dispersion et ensuite le refroidissement et la collecte des particules revêtues, ainsi obtenues.

7. Composition pharmaceutique pour une administration orale à des animaux à sang chaud en particulier à des chats, des chiens et des chevaux, comprenant
f) une quantité efficace sur le plan vétérinaire d'une composition selon la revendication 1,
g) des quantités efficaces sur le plan vétérinaire d'un ou plusieurs agents anti-nématode choisis parmi des benzimidazoles, des tétrahydropyrimidines ou des lactones macrocycliques telles que des avermectines, des milbémycines et des dérivés correspondants,
h) un ou plusieurs excipients physiologiquement acceptables.

8. Composition pharmaceutique selon la revendication 7 sous la forme de comprimés et de pâtes orales ou gels oraux.

9. Composition pharmaceutique selon la revendication 7 contenant du praziquantel dans la plage de 0,5 à 25 % p/p, sur la base de la formulation entière.

10. Composition pharmaceutique selon la revendication 7, l'agent de type benzimidazole étant le fébantel ou le fenbendazole et le composé de type tétrahydropyrimidine étant l'embonate de pyrantel.

11. Composition pharmaceutique selon la revendication 7, la lactone macrocyclique étant de formule : 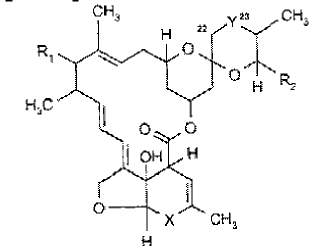 X étant -C(H)(OH)- ; -C(O)- ; ou -C(=N-OH)- ; Y étant - C(H2)- ; =C(H)- ; -C(H) (OH)- ; ou - C(=N-OCH3)- ; R-i étant hydrogène ou l'un parmi les radicaux 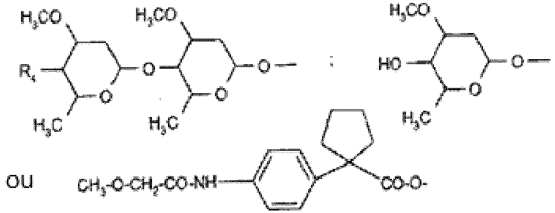 R4 étant hydroxyle, -NH-CH3 or-NH-OCH3; et R2 étant hydrogène, -CH3, -C2H5, -CH(CH3)2, - CH(CH3)-C2H5, - C(CH3)=CH-CH(CH3)2 ou cyclohexyle ; et si la liaison entre les atomes 22 et 23 représente une double liaison l'atome de carbone en position 23 est non substitué de sorte que Y est =C(H)-, ou si la liaison entre les atomes 22 et 23 est une simple liaison l'atome de carbone en position 23 est non substitué ou substitué par hydroxy ou par le groupe =N-O-CH3 de sorte que Y est -C(H2)- ; - C(H)(OH)- ; ou -C(=N-OCH3)- ; sous forme libre ou sous la forme d'un sel physiologiquement acceptable.

12. Composition pharmaceutique selon la revendication 7 contenant un ou plusieurs agents anti-nématode dans la plage de 0,01 à 50 % p/p, sur la base de la formulation entière.
